# EUROPÄISCHE PATENTSCHRIFT

(11) **EP 2 683 517 B1**
(45) Veröffentlichungstag und Bekanntmachung des Hinweises auf die Patenterteilung: **16.09.2015**
(21) Anmeldenummer: 13703522.6
(22) Anmeldetag: 22.01.2013
(51) Int. Cl.: A61B 17/221, A61B 17/00, B23K 26/36, A61F 2/01, A61F 2/91, B23K 26/06

(54) **VERFAHREN ZUM HERSTELLEN EINES KÖRPERIMPLANTATS, BAUGRUPPE AUS EINEM FÜHRUNGSDRAHT UND EINEM KÖRPERIMPLANTAT SOWIE MEDIZINISCHES INSTRUMENT**
METHOD FOR PRODUCING A BODY IMPLANT, ASSEMBLY CONSISTING OF A GUIDE WIRE AND A BODY IMPLANT, AND A MEDICAL INSTRUMENT
PROCÉDÉ DE FABRICATION D'UN IMPLANT CORPOREL, MODULE CONSTITUÉ D'UN FIL-GUIDE ET D'UN IMPLANT CORPOREL ET INSTRUMENT MÉDICAL

(30) Priorität: 30.05.2012 DE 102012010687
(43) Veröffentlichungstag der Anmeldung: 15.01.2014
(73) Patentinhaber: Admedes Schuessler GmbH, 75179 Pforzheim (DE)
(72) Erfinder: FETH, Nils-Agne, 76337 Waldbronn (DE); LANGE, Alexander, 76135 Karlsruhe (DE); STEINER, Ralf, 75173 Pforzheim (DE)
(74) Vertreter: Müller-Boré & Partner Patentanwälte PartG mbB
(86) Internationale Anmeldenummer: PCT/EP2013/000185
(87) Internationale Veröffentlichungsnummer: WO 2013/178297

(56) Entgegenhaltungen:
- DE-A1- 19 722 429
- DE-A1- 19 745 294
- US-A1- 2002 065 553
- US-A1- 2010 102 046

## Beschreibung

Die vorliegende Erfindung bezieht sich auf ein Verfahren zum Herstellen eines Körperimplantats, eine Baugruppe aus einem Führungsdraht und einem Körperimplantat sowie auf ein medizinisches Instrument.

Herkömmlich werden Körperimplantate sowie medizinische Instrumente, wie beispielsweise Stents, Körbchen, Filter oder Catching Devices, aus einem hohlen Rundmaterial, wie beispielsweise einem Rohr, hergestellt. Alternativ oder zusätzlich können diese Vorrichtungen durch Flechten von Drähten hergestellt werden. Dabei ist ein Fertigungsaufwand jedoch hoch und eine Crimpfähigkeit, das heißt eine Fähigkeit einer Durchmesserverringerung zum Einführen in den menschlichen Körper, ist bei derart hergestellten Vorrichtungen begrenzt. Es besteht somit ein Bedarf für ein Verfahren und durch dieses Verfahren hergestellte Vorrichtungen, die eine höhere Crimpfähigkeit aufweisen und einfacher herstellbar sind.

DE 197 22 429 A1 offenbart eine Vorrichtung zum Einfangen und/oder Zerkleinern von Gegenständen in Hohlorganen, die aus einem Zugstrang hergestellt wird, indem mittels Laserstrahl schlitzförmige Öffnungen erzeugt werden.

Die Aufgabe der Erfindung besteht somit in der Schaffung eines vereinfachten Verfahrens sowie entsprechender Vorrichtungen, die die Herstellung von Stents, Körbchen, Filter und dergleichen vereinfachen und die eine höhere Crimpfähigkeit ermöglichen.

Diese Aufgabe wird durch die Merkmale der unabhängigen Ansprüche gelöst. Vorteilhafte Weiterbildungen der Erfindung sind in den abhängigen Ansprüchen definiert.

Gemäß einem Gesichtspunkt betrifft die Erfindung ein Verfahren zum Herstellen eines Körperimplantats mit den Schritten:
Bereitstellen eines Drahts;
Erzeugen von Schnitten bzw. Einkerbungen bzw. Strukturen im Querschnitt des Drahts mittels Ultrakurzpulslaser, um eine vorgegebene Gestalt des Körperimplantats zu erzeugen. Durch die Schnitte wird ein Teil des Querschnitts des Drahts entfernt, um dadurch ein Gelenk zu erzeugen.

Vorzugsweise werden die Schnitte im wesentlichen in radialer Richtung erzeugt und ein geschnittener Abschnitt des Drahts oder ein Drahtsegment wird aufgeweitet, um ein Körperimplantat, wie beispielsweise ein geschlossenes Körbchen oder ein Filter, Stent oder dergleichen zu erzeugen.

Durch die Schnitte kann der größte Teil, des Querschnitts des Drahts entfernt werden, um dadurch ein Gelenk zu erzeugen.

Weiter bevorzugt kann das Verfahren des weiteren den Schritt des Verflechtens von Drahtsegmenten und/oder einen Schritts einer Formgebung von Drahtsegmenten aufweisen.

Vorzugsweise wird ein Draht aus einem Material mit Formgedächtniseigenschaften, wie beispielsweise Nitinol, verwendet.

Die Ultrakurzpuls-Lasertechnologie (UKP) ermöglicht die Mikrobearbeitung von Materialien, wie beispielsweise Drähten ohne die Notwendigkeit, das entfernte Material, wie beispielsweise wiedererstarrte Schmelze beim konventionellen Laser-Schmelzschneiden auszutreiben. Es findet also ein kalter Abtragungsprozeß, ein sogenannter Ablationsprozeß statt. Im Gegensatz zum konventionellen Laser-Schmelzschneiden, bei dem ausschließlich durchgehende Schnitte möglich sind, ergibt sich die Möglichkeit, einen Draht einerseits zu strukturieren und damit die mechanischen und/oder elektrischen bzw. elektronischen Eigenschaften zu verändern. Andererseits erlaubt dieser Prozeß sehr feine Drähte symmetrisch und asymmetrisch durch Schnitte aufzuschlitzen. Durch Erzeugen von radialen Schnitten in dem Draht können somit Drahtsegmente von dem Draht gelöst werden und anschließend geformt und/oder aufgeweitet werden, um beispielsweise ein Körbchen, einen Stent oder einen Filter zu erzeugen. Darüber hinaus können die Drahtsegmente verflochten werden, um mechanische und elektrische bzw. elektronische Eigenschaften gezielt zu beeinflussen.

Darüber hinaus können die Schnitte bzw. Einkerbungen bzw. Strukturen so erzeugt werden, daß ein einstückig angeformtes Gelenk aus dem Draht erzeugt wird, indem ein großer Teil des Querschnitts entfernt wird. Somit ergeben sich vielzählige Gestaltungsmöglichkeiten, indem an einem Draht entsprechende Schnitte mit der Ultrakurzpuls-Lasertechnologie erzeugt werden.

Gemäß einem weiteren Gesichtspunkt betrifft die Erfindung eine Baugruppe aus einem Führungsdraht und einem Körperimplantat, die durch Bereitstellen eines Drahts und Erzeugen von vorgegebenen Schnitten an dem Draht mittels Ultrakurzpulslaser hergestellt ist, wobei Führungsdraht und Körperimplantat einstückig bzw. monolithisch ausgebildet sind und eine Sollbruchstelle aufweisen. Dabei kann der Führungsdraht zumindest ein einstückig ausgebildetes Gelenk aufweisen, um eine Flexibilität bzw. Biegsamkeit des Führungsdrahts zu erhöhen.

Vorzugsweise weist die Baugruppe zumindest eines aus einem Stent, einem Körbchen, oder einem Filter auf.

Gemäß einem weiteren Gesichtspunkt wird ein medizinisches Instrument mit einem Führungsdraht zur Verfügung gestellt, das durch Bereitstellen eines Drahts und Erzeugen von vorgegebenen Schnitten an dem Draht mittels Ultrakurzpulslaser hergestellt ist. Dabei weist der Führungsdraht zumindest ein einstückig ausgebildetes Gelenk auf.

Diese Fertigungstechnologie hat den Vorteil, daß eine Baugruppe aus einem Führungsdraht und einem Körperimplantat bzw. einem medizinischen Instrument aus einem Stück herstellbar ist, so daß ein Verbinden beispielsweise durch Mikroschweißen eines Führungsdrahts mit einem Körperimplantat nicht mehr durchgeführt werden muß. Somit wird eine Fehleranfälligkeit einer entsprechenden Baugruppe aus einem Führungsdraht und einem medizinischen Instrument wesentlich verringert.

Diese einstückig ausgebildete Baugruppe kann eine Sollbruchstelle aufweisen, so daß das Körperimplantat bzw. medizinische Instrument nach dem Plazieren in dem menschlichen Körper an der Sollbruchstelle von dem Führungsdraht getrennt wird, um den Führungsdraht zu entfernen. Des weiteren ist der Herstellungsprozess stark vereinfacht. Darüber hinaus kann der Führungsdraht ein einstückig ausgebildetes Gelenk aufweisen, um eine höhere Flexibilität aufzuweisen.

Diese Technologie hat den Vorteil von geringeren Kosten für das Rohmaterial, ist mechanisch stabiler und hat eine maximale Crimpfähigkeit, weil die aufgeweiteten Drahtsegmente bis zur ursprünglichen Lage des nicht geschnittenen Drahts verformt bzw. gecrimpt werden können, um den Durchmesser zum Einführen in den menschlichen Körper auf einen minimalen Durchmesser zu verringern. Darüber hinaus kann die Vorrichtung mit einer minimalen Anzahl von Fertigungsschritten erzeugt werden.

Die Erfindung wird nun anhand von Ausführungsbeispielen unter Bezugnahme auf die Zeichnungen näher erläutert.
Fig. 1 zeigt einen kreisrunden Draht, der durch einen Laserstrahl in radialer Richtung in etwa bis zu dessen Mitte geschnitten wird.
Fig. 2 zeigt das Erzeugen von insgesamt fünf Schnitten in einer radialen Richtung bis zur Mitte des Drahts.
Fig. 3 zeigt ein Körbchen oder Filterdesign, das durch die Schnitte erzeugt wird, indem die abgeschnittenen Drahtsegmente anschließend aufgeweitet werden.
Fig. 4 zeigt einen Führungsdraht, der durch Erzeugen entsprechender Schnitte eine hohe Biegsamkeit bzw. Flexibilität aufweist.
Fig. 5 zeigt einen Stent, der einstückig mit dem Führungsdraht hergestellt ist und eine Sollbruchstelle aufweist.
Fig. 6 zeigt einen steuerbaren Führungsdraht.
Fig. 7 zeigt eine Detailansicht des Führungsdrahts von Fig. 6 mit einem entsprechenden Gelenk zum Abknicken des Führungsdrahts.
Fig. 8a zeigt ein Drahtsegmente, das als Kreisausschnitt geformt ist, und Fig. 8b zeigt ein Drahtsegment, das als Kreisabschnitt geformt ist.

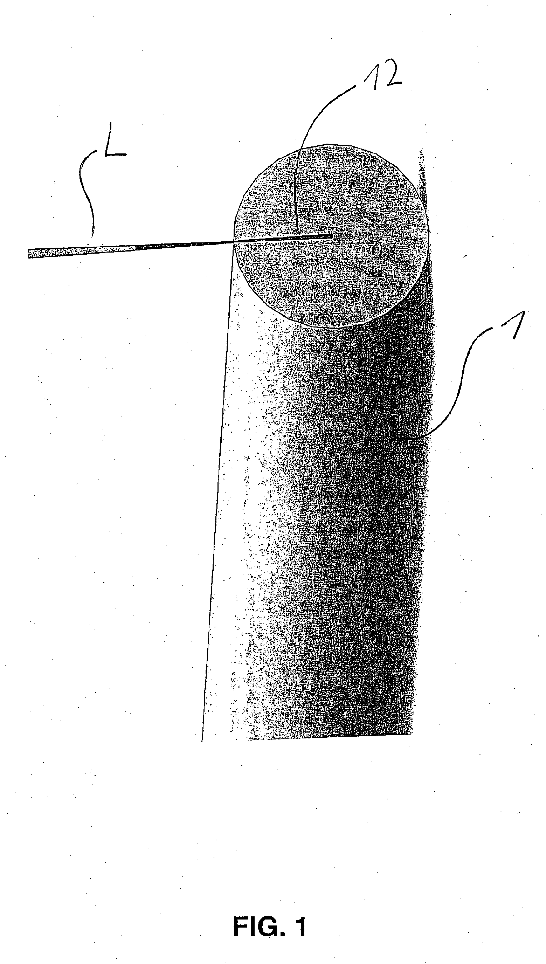

Wie in Fig. 1 gezeigt ist, wird ein kreisrunder Draht 1 durch einen Laserstrahl L mittels der Ultrakurzpuls-Lasertechnologie bis etwa zu dessen Mitte in radialer Richtung eingeschnitten, um einen Schnitt 12 zu erzeugen. Obwohl es vorteilhaft ist, kreisrunde Drähte für diese Technologie anzuwenden, ist die Erfindung nicht darauf beschränkt. Es können auch Drähte mit elliptischem Querschnitt, Dreikantdrähte, Vierkantdrähte, Fünfkantdrähte oder dergleichen verwendet werden.

Vorteilhafterweise wird dabei ein Ultrakurzpuls-Laser mit einer Wellenlänge von etwa 200 bis etwa 2000 nm eingesetzt. Die Impulsdauer sollte etwa 10 fs bis etwa 10 ps betragen.

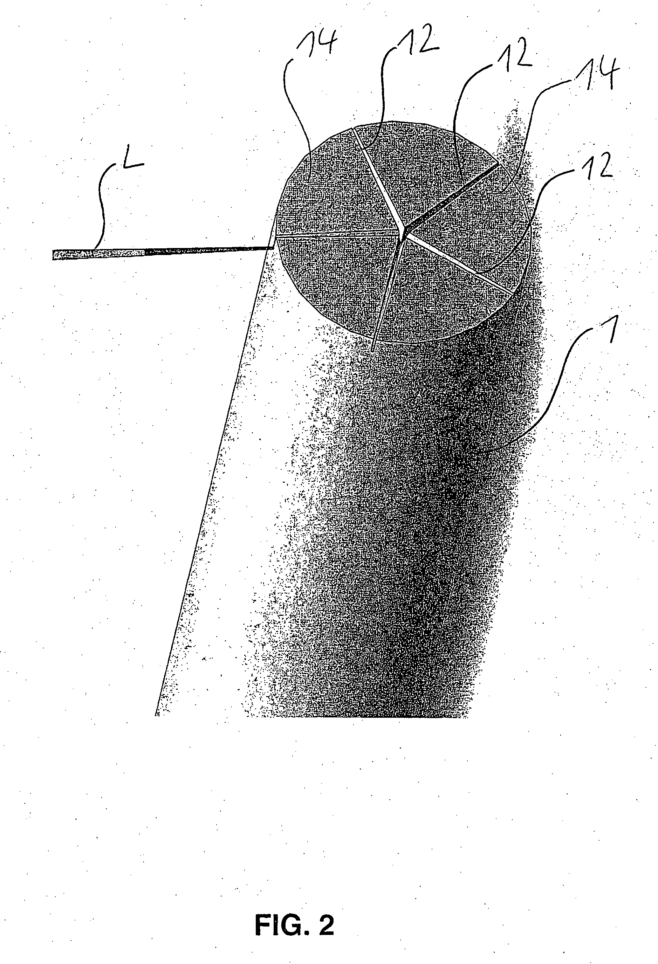

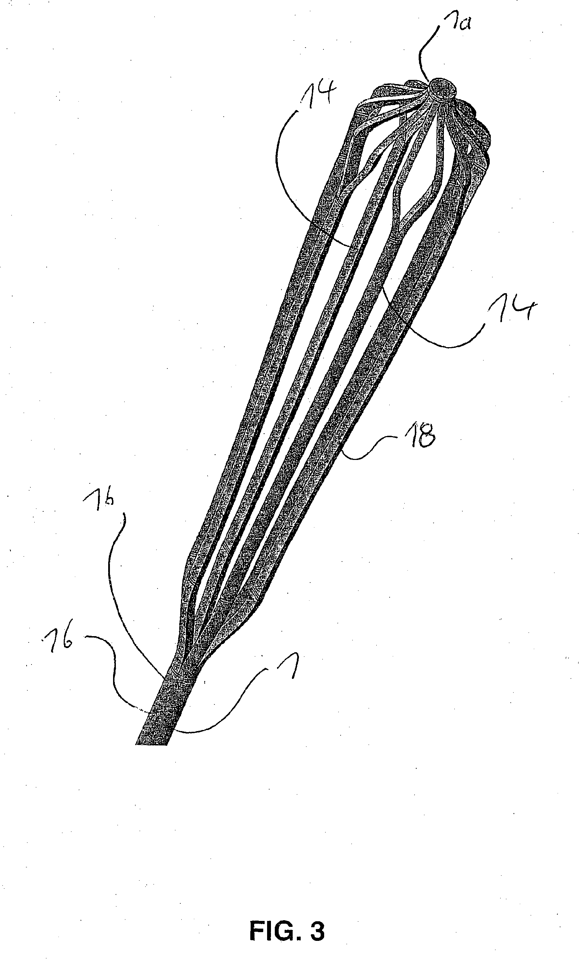

Wie des weiteren in Fig. 2 gezeigt ist, können beispielsweise fünf radiale Schnitte bis zur Mitte des Drahts 1 erzeugt werden, um Drahtsegmente oder geschnittene Abschnitte 14 zu erzeugen. Diese geschnittenen Abschnitte oder Drahtsegmente 14 können anschließend durch Verformen aufgeweitet werden, um beispielsweise ein Körbchen oder Filterdesign zu erzeugen, wie es beispielsweise in Fig. 3 gezeigt ist. Dabei gehen die Schnitte nicht bis zu einem axialen Ende 1 a, 1 b des Drahts 1, so daß die einzelnen Drahtsegmente 14 an ihren Enden in Längsrichtung noch miteinander verbunden sind.

Auf diese Weise wird aus einem Draht 1 ein Körbchen bzw. Filter erzeugt, das Drahtsegmente 14 an den Enden 1 a, 1b einstückig oder integral verbunden aufweist.

In anderen Worten wird der Draht 1 wie ein Kuchen in einzelne Segmente (Drahtsegmente 14) aufgetrennt, wobei axiale Enden 1 a, 1 b des Drahts nicht getrennt werden. Die aufgetrennten Drahtsegmente 14 werden aufgeweitet, so dass ein Körper mit beabstandeten Drahtsegmenten 14 in der axialen Mitte und einstückig mit den Drahtsegmenten 14 verbundenen axialen Enden 1 a, 1 b geformt wird.

Es versteht sich, dass die Erfindung auch mit drei, vier, sechs oder sieben Schnitten 12 etc. ausgeführt werden kann. Dabei müssen die Schnitte 12 nicht in gleichmässigen Winkelabständen durchgeführt werden, sondern es können unterschiedlich große "Kuchenstücke" bzw. Drahtsegmente 14 erzeugt werden.

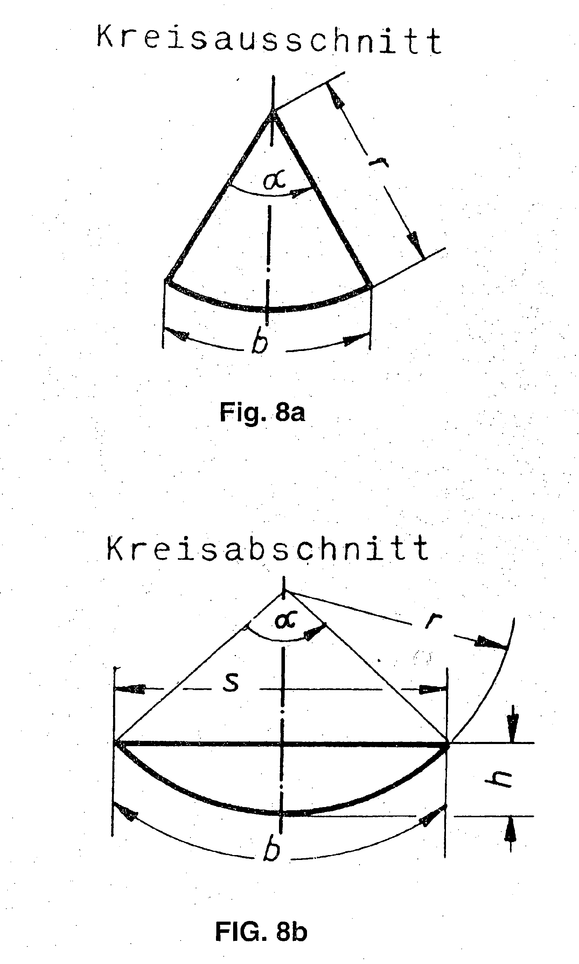

Darüber hinaus müssen die Drahtsegmente 14 nicht als Kreisausschnitte mit der Länge des Radius r des Drahts 1 als Seiten des Kreisausschnitts erzeugt werden, d.h. durch Schnitte 12, die im Mittelpunkt des Drahtquerschnitts enden, wie in Fig. 8a gezeigt ist, sondern es können beliebige Formen, wie beispielsweise ein Kreisabschnitt von dem Drahtquerschnitt getrennt werden, wie beispielsweise in Fig. 8b gezeigt ist.

Ein Kreisabschnitt, wie in Fig. 8b gezeigt ist, wird dadurch von dem Drahtquerschnitt herausgetrennt, dass ein Schnitt 12 versetzt zu dem Mittelpunkt des Drahtquerschnitts entlang der Sehnenlänge s erzeugt wird. Dabei ist eine Höhe h des Schnitts 12 geringer als der Radius r des Drahts.

Indem auf diese Weise Drahtsegmente 14 mit unterschiedlichen Querschnitten von dem Drahtquerschnitt getrennt und anschließend aufgeweitet werden, kann beispielsweise ein Stent erzeugt werden, der unterschiedliche Stützkräfte entlang seines Umfangs aufweist.

Diese Drahtsegmente 14 können einer weiteren Formgebung durch Umformen oder weitere Laserbearbeitung entsprechend den gewünschten mechanischen und/oder elektrischen Eigenschaften verformt oder bearbeitet werden. Darüber hinaus können die Drahtsegmente 14 verflochten werden, beispielsweise.durch Klöppeln der Drahtsegmente mit den Bindungsarten 1/2, 1/1 oder 2/2.

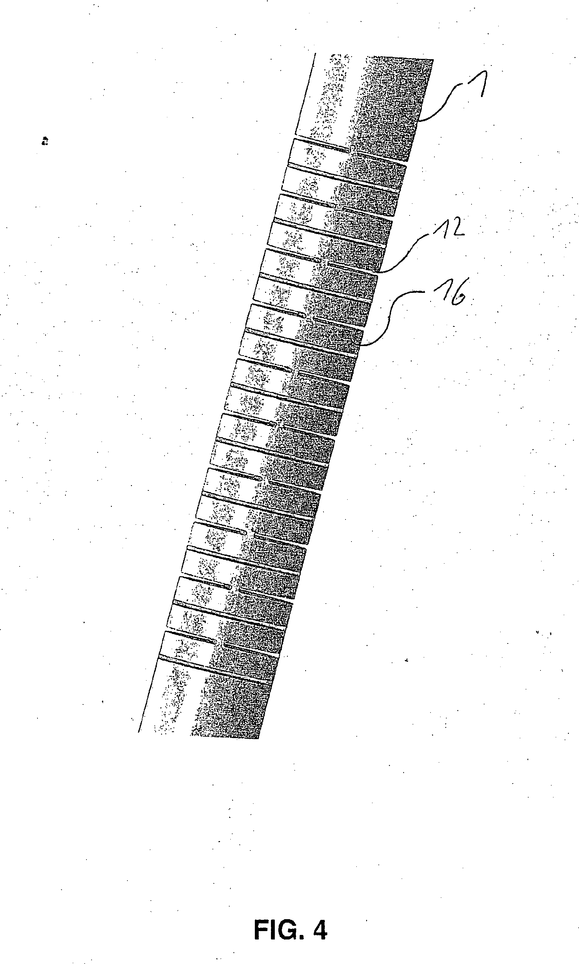

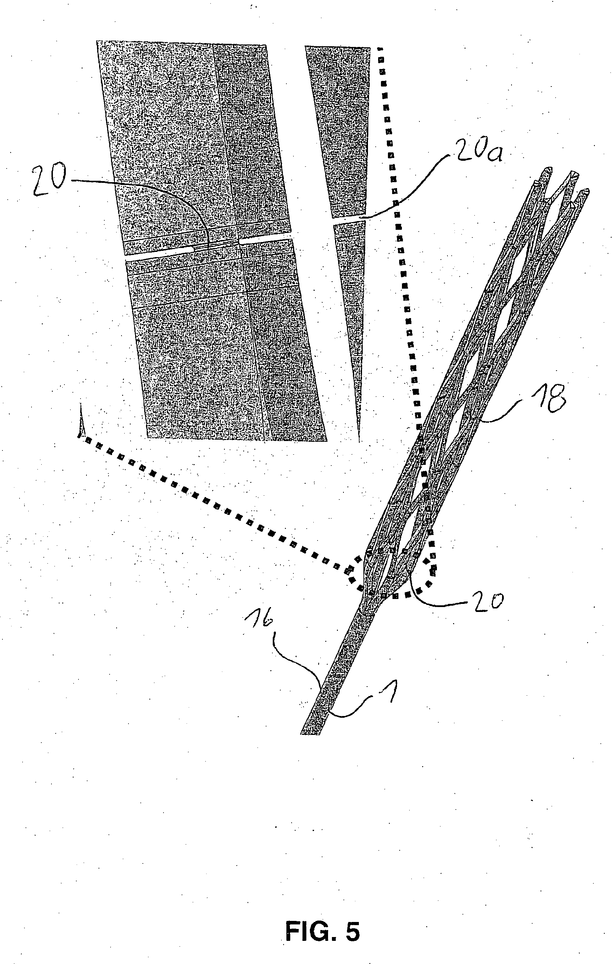

Das somit erzeugte Körbchen oder Filterdesign bzw. Stent 18 kann anschließend vom Draht 1 getrennt werden. Es kann jedoch auch an einem längeren Stück Draht 1 verbleiben, so daß das nicht geschnittene Ende des Drahts 1 als Führungsdraht 16 verwendet wird, wie beispielsweise in Figs. 4 und 5 gezeigt ist.

Zwischen dem Führungsdraht 16 und dem erzeugten Körperimplantat 18 kann sich dabei eine Sollbruchstelle 20 befinden, so daß die Baugruppe aus dem Führungsdraht 16 und dem Körperimplantat 18, die auf diese Weise einstückig erzeugt wird, nach dem Einführen und Plazieren des Körperimplantats 18 an der Sollbruchstelle 20 getrennt werden kann, um den Führungsdraht 16 nach dem Plazieren des Körperimplantats 18 zu entfernen. Der Führungsdraht 16 kann dabei wie in Fig. 4 gezeigt ist, entsprechende Schnitte 12 bzw. Querschnittsschwächungen bzw. Strukturen aufweisen, um eine hohe Biegsamkeit bzw. Flexibilität zu erzeugen. Die Sollbruchstelle 20 hat eine entsprechende Querschnittsschwächung 20a, um ein Abbrechen oder Ablösen an der Sollbruchstelle 20 zu erleichtern.

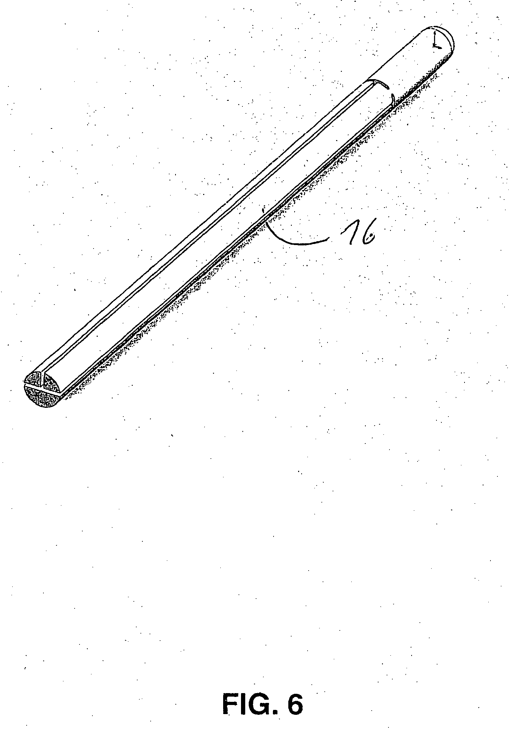

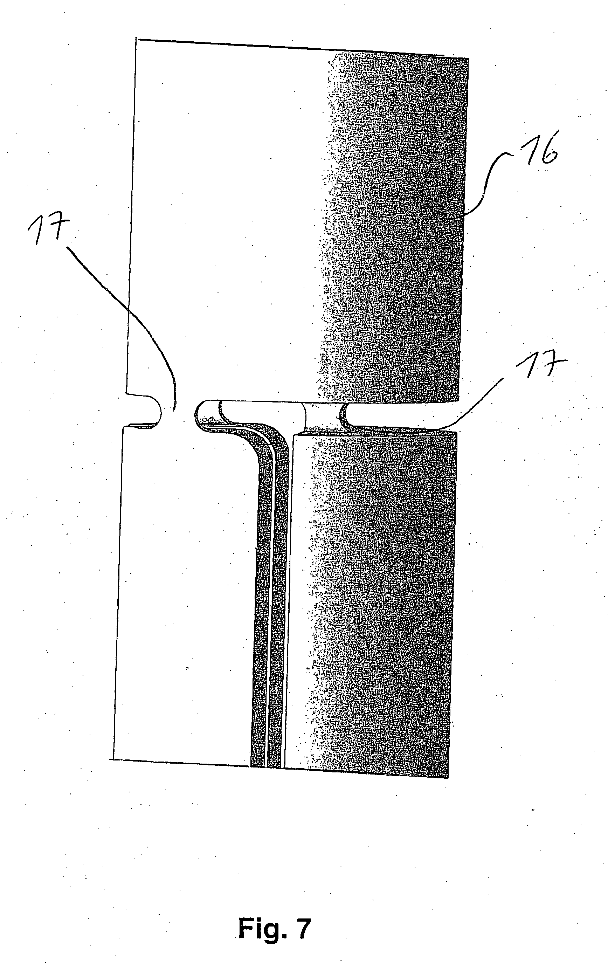

Wie des weiteren in den Figuren 6 und 7 gezeigt ist, kann durch einen Schnitt, bei dem fast der gesamte Querschnitt des Drahts bzw. Führungsdrahts 16 entfernt wird, ein entsprechendes Gelenk 17 erzeugt werden, um das ein Ende des Führungsdrahts 16 gegenüber einem anderen Abschnitt gebogen werden kann.

Es ergeben sich somit vielfältige Designmöglichkeiten, indem ein Draht 1 durch die UKP-Lasertechnologie entsprechend geschnitten wird und die geschnittenen Drahtsegmente 14 aufgeweitet und eventuell weiter bearbeitet werden. Auf diese Weise kann ein Körperimplantat 18, wie beispielsweise ein Stent, ein Körbchen oder ein Filter, einstückig auf einfache Weise hergestellt werden. Darüber hinaus kann dieses Körperimplantat 18 bis zum ursprünglichen Drahtdurchmesser gecrimpt werden, um eine maximale Durchmesserverringerung zu erzielen.

Vorzugsweise wird ein Draht 1 aus einem Material mit Formgedächtniseigenschaften wie beispielsweise Nitinol verwendet. Es können jedoch auch andere Metalle oder auch Nichtmetalle verwendet werden.

Die Erfindung ist nicht auf das Erzeugen von radialen Schnitten 12 beschränkt, die Schnitte können auch in anderen Richtungen erzeugt werden. Darüber hinaus müssen die Schnitte 12 nicht bis zur Mitte des Drahts 1 gehen, sondern können, je nach Anwendungsfall, weniger tief oder tiefer erzeugt werden. Hiedurch können Strukturen oder Einkerbungen an dem Querschnitt des Drahts 1 erzeugt werden.

### Bezugszeichenliste

- 1: Draht
- 12: Schnitt
- 14: Drahtsegmeht.
- 16: Führungsdraht
- 17: Gelenk
- 18: Körperimplantät (Stent)
- 20: Sollbruchstelle
- 20a: Querschnitttsschwächung
- L: Laser

## Patentansprüche

1. Verfahren zum Herstellen eines Körperimplantats (18) oder medizinischen Instruments mit den Schritten:
Bereitstellen eines Drahts (1);
Erzeugen von vorgegebenen Schnitten (12) an dem Draht (1) mittels Ultrakurzpulslaser (L), um eine vorgegebene Gestalt des Körperimplantats (18) zu erzeugen, wobei durch die Schnitte (12) ein Teil des Querschnitts des Drahts (1) entfernt wird, um ein Gelenk (17) zu erzeugen.

2. Verfahren nach Anspruch 1, wobei die Schnitte (12) im wesentlichen in radialer Richtung erzeugt werden und ein geschnittener Abschnitt (14) des Drahts (1) oder ein Drahtsegment aufgeweitet wird, um ein geschlossenes Körbchen oder ein Filter zu erzeugen.

3. Verfahren nach einem der vorherigen Ansprüche, des weiteren mit dem Schritt des Verflechtens bzw. des Klöppelns von Drahtsegmenten und/oder eines Schritts einer Formgebung von Drahtsegmenten.

4. Verfahren nach einem der vorherigen Ansprüche, wobei ein Draht (1) aus einem Material mit Formgedächtniseigenschaften verwendet wird.

5. Baugruppe aus einem Führungsdraht (16) und einem Körperimplantat (18) oder medizinischen Instrument, die durch Bereitstellen eines Drahts (1) und Erzeugen von vorgegebenen Schnitten (12) an dem Draht (1) mittels Ultrakurzpulslaser (L), um eine vorgegebene Gestalt des Körperimplantats (18) zu erzeugen, hergestellt ist, wobei Führungsdraht (16) und Körperimplantat (18) einstückig ausgebildet sind und eine Sollbruchstelle (20) aufweisen.

6. Baugruppe nach Anspruch 5, wobei der Führungsdraht (1) zumindest ein ein stückig ausgebildetes Gelenk (20) aufweist.

7. Baugruppe nach einem der Ansprüche 5 oder 6, wobei das Körperimplantat (18) oder das medizinische Instrument zumindest eines aus einem Stent, einem Körbchen, oder einem Filter aufweist.

8. Medizinisches Instrument mit einem Führungsdraht (16), das durch Bereitstellen eines Drahts (1) und Erzeugen von vorgegebenen Schnitten (12) an dem Draht (1) mittels Ultrakurzpulslaser (L) hergestellt ist, wobei der Führungsdraht (16) zumindest ein einstückig ausgebildetes Gelenk (20) aufweist.

## Claims

1. A method for producing a body implant (18) or medical instrument comprising the steps:
providing a wire (1);
producing predetermined cuts (12) on the wire (1) by means of an ultra-short pulse laser (L) in order to produce a predetermined shape of the body implant (18), wherein a part of the cross section of the wire (1) is removed by the cuts (12) in order to produce a joint (17).

2. The method according to claim 1, wherein the cuts (12) are produced substantially in the radial direction and a cut portion (14) of the wire (1) or a wire segment is widened in order to produce a closed basket or a filter.

3. The method according to one of the preceding claims, further comprising the step of interweaving or respectively braiding wire segments and/or a step of shaping wire segments.

4. The method according to one of the preceding claims, wherein a wire (1) made of a material having shape memory properties is used.

5. An assembly consisting of a guide wire (16) and a body implant (18) or medical instrument which is produced by providing a wire (1) and producing predetermined cuts (12) on the wire (1) by means of an ultra-short pulse laser (L) in order to produce a predetermined shape of the body implant (18), wherein guide wire (16) and body implant (18) are configured in one piece and have a predetermined rupture point (20).

6. The assembly according to claim 5, wherein the guide wire (1) has at least one joint (20) configured in one piece.

7. The assembly according to one of claims 5 or 6, wherein the body implant (18) or the medical instrument has at least one of the following: a stent, a basket or a filter.

8. A medical instrument having a guide wire (16) which is produced by providing a wire (1) and producing predetermined cuts (12) on the wire (1) by means of an ultra-short pulse laser (L), wherein the guide wire (16) has at least one joint (20) which is configured in one piece.

## Revendications

1. Procédé de fabrication d'un implant corporel (18) ou d'un instrument médical avec les étapes suivantes consistant à :
fournir un fil (1) ;
produire de coupes (12) prédéterminées dans le fil (1) au moyen d'un laser à impulsions ultracourtes (L), pour créer une forme prédéterminée de l'implant corporel (18), sachant que par les coupes (12) une partie de la section transversale du fil (1) est éliminée, pour créer une articulation (17).

2. Procédé d'après la revendication 1, sachant que les coupes (12) sont effectuées essentiellement en direction radiale et qu'une portion coupée (14) du fil (1) ou un segment de fil est élargi, pour créer une cage (*Körbchen*) fermée ou un filtre.

3. Procédé-d'après une des revendications précédentes, comprenant en outre l'étape consistant à entrelacer ou encore broder des segments de fil et/ou une étape de façonnage de segments de fil.

4. Procédé d'après une des revendications précédentes, sachant qu'on utilise un fil (1) constitué d'un matériau présentant des propriétés de mémoire de forme.

5. Module constitué d'un fil-guide (16) et d'un implant corporel (18) ou d'un instrument médical, qui est fabriqué par la mise à disposition d'un fil (1) et la production de coupes (12) prédéterminées dans le fil (1) au moyen d'un laser à impulsions ultracourtes (L), pour créer une forme prédéterminée de l'implant corporel (18), sachant que le fil-guide (16) et l'implant corporel (18) sont réalisés d'une seule pièce et présentent une zone de rupture imposée (20).

6. Module d'après la revendication 5, sachant que le fil-guide (1) présente au moins une articulation (20) réalisée d'une seule pièce.

7. Module d'après une des revendications 5 ou 6, sachant que l'implant corporel (18) ou l'instrument médical présente au moins un parmi les suivants : un stent, une cage (*Körbchen*) ou un filtre.

8. Instrument médical avec un fil-guide (16), qui est fabriqué par la mise à disposition d'un fil (1) et la production de coupes (12) prédéterminées dans le fil (1) au moyen d'un laser à impulsions ultracourtes (L), sachant que le fil-guide (16) présente au moins une articulation (20) réalisée d'une seule pièce.
